# FASCICULE DE BREVET EUROPEEN

(11) **EP 2 134 681 B1**
(45) Date de publication et mention de la délivrance du brevet: **16.05.2012**
(21) Numéro de dépôt: 08718147.5
(22) Date de dépôt: 21.03.2008
(51) Int. Cl.: C07D 207/36, C07D 215/38, C07D 491/048

(54) **REARRANGEMENT TRANSANNULAIRE DE LACTAMES ACTIVES**
TRANSANNULARE UMLAGERUNG VON AKTIVEN LAKTAMEN
TRANS-ANNULAR REARRANGEMENT OF ACTIVE LACTAMES

(30) Priorité: 21.03.2007 FR 0753973
(43) Date de publication de la demande: 23.12.2009
(73) Titulaire: Centre National de la Recherche Scientifique (CNRS), 75016 Paris (FR); Université de Montpellier I, 34006 Montpellier (FR)
(72) Inventeur: MARTINEZ, Jean, F-34720 Caux (FR); DEWYNTER, Georges, F-34090 Montpellier (FR); FARRAN, Daniel, F-34800 Cabrieres (FR)
(74) Mandataire: Warcoin, Jacques
(86) Numéro de dépôt international: PCT/EP2008/053449
(87) Numéro de publication internationale: WO 2008/125421

(56) Documents cités:
- DE-A1- 1 934 383
- FR-A- 2 320 752
- POTHION C ET AL: "Synthesis of Pyrrolidine-2,4-diones from Urethane N-carboxyanhydrides (UNCAs)" TETRAHEDRON LETTERS, ELSEVIER, AMSTERDAM, NL, vol. 37, no. 7, 12 février 1996 (1996-02-12), pages 1027-1030, XP004030219 ISSN: 0040-4039

## Description

L'invention concerne un nouveau procédé permettant de synthétiser des produits naturels, ou des intermédiaires chiraux de synthèse de produits naturels, de façon simple et stéréocontrôlée.

Ce procédé donne accès à une série de molécules dont les voies de synthèse existantes sont longues et fastidieuses.

En effet, les procédés de l'art antérieur de dimérisation des UNCAs sont toujours racémisants et n'autorisent aucune diversité structurale des composés obtenus puisque les groupes R des chaînes latérales sont par définitions identiques. Or de façon surprenante, le réarrangement du procédé selon la présente invention est totalement énantiocontrôlé et les produit s obtenus peuvent être optiquement purs. La synthèse de dérivés de pyrrolidine -2,4-dione et de dihydroquinoline-2,4-dione a déjà été décrite dans DE 1 934 383 et FR 2 320 752.

Le procédé selon l'invention met en oeuvre un réarrangement de lactames activés tel que les dicétopipérazines et les benzodiazépinediones en conditions basiques.

Le procédé de l'invention permet de préparer des aminotetramates et des aminoquinoléines, par une étape de réduction de cycle concomitante à une exclusion d'un groupe amino, et éventuellement une substitution simultanée du cycle.

L'invention concerne également une nouvelle famille de composés d'intérêt biologique ou pharmaceutique, pouvant être obtenus selon le procédé de l'invention.

Plus précisément, l'invention concerne un procédé de synthèse d'un composé de formule (I) suivante : 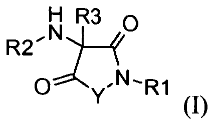 dans laquelle :
- R1 et R2 représentent indépendamment l'un de l'autre un groupe N-protecteur;
- R3 représente un atome d'hydrogène, un groupe alkyle en C₁ à C₆, un groupe aryl(alkyle en C₁ à C₆), un groupe alcényle en C₂ à C₆, ou un groupe alkoxycarbonylalkyle ;
- Y représente
   un groupe -C(HR4)- dans lequel R4 représente l'atome d'hydrogène, un groupe alkyle en C₁ à C₆, un groupe aryle, un groupe aryl(alkyle en C₁ à C₆), ou un groupe alcényle en C₂ à C₆ ou
   un groupe ortho-phénylène,
   caractérisé en ce qu'il comprend :
- une étape (a₁) consistant à faire réagir avec une base le composé de formule (II) suivante : 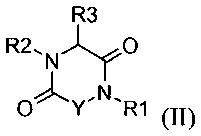 dans laquelle R1, R2, R3 et Y sont tels que définis précédemment, ou,
- une étape (a₂) consistant à faire réagir avec une base le composé de formule (III) suivante : 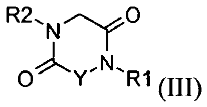 en présence d'un composé R3-X dans lesquelles R1 et R2 sont tels que définis précédemment, R3 est tel que défini précédemment à l'exclusion de l'hydrogène, et X représente un halogène,
   ou,
- dans le cas où Y représente un groupe -C(HR4)-, une étape (a₃) consistant à faire réagir avec une base le composé de formule (IV) suivante : 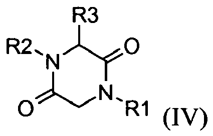 en présence d'un composé de formule R4-X dans lesquelles R1, R2, R3 sont tels que définis précédemment, R4 est tel que défini précédemment à l'exclusion de l'hydrogène, et X représente un halogène,
   ou
- dans le cas où Y représente un groupe -C(HR4)-, une étape (a₄) consistant à faire réagir avec une base le composé de formule (V) suivante : 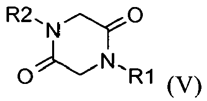
en présence d'un composé de formule R4-X, dans lesquelles R1 et R2 sont tels que définis précédemment, R3 est identique à R4 et est tel que défini précédemment à l'exclusion de l'hydrogène, et X représente un halogène.

X est avantageusement Cl ou Br.

Par le terme « groupe alkyle en C₁ à C₆ », on entend au sens de la présente invention tout groupe alkyle de 1 à 6 atomes de carbones, linéaire ou ramifié, en particulier, les groupes méthyle, éthyle, *n*-propyle, isopropyle, *n*-butyle, *iso-*butyle, *sec*-butyle, *t*-butyle, *n*-pentyle, *n*-hexyle. Avantageusement il s'agit d'un groupe méthyle ou isopropyle.

Par le terme « groupe alcényle C₂ à C₆ », on entend au sens de la présente invention tout groupe alcényle de 2 à 6 atomes de carbones, linéaire ou ramifié, en particulier le groupe vinyle ou allyle, avantageusement un allyle.

Par le terme « groupe aryle », on entend au sens de la présente invention un ou plusieurs cycles aromatiques ayant 5 à 8 atomes de carbones, pouvant être accolés ou fusionnés. En particulier, les groupes aryles peuvent être des groupes monocycliques ou bicycliques, de préférence phényle, naphthyle, tetrahydronaphthyle ou indanyle. Avantageusement il s'agit d'un groupe phényle.

Par le terme « groupe arylalkyle en C₁ à C₆ », on entend au sens de la présente invention tout groupe aryle tel que défini ci-dessus, lié par l'intermédiaire d'un groupe alkyle en C₁ à C₆ tel que défini ci-dessus. En particulier un groupe arylalkyle est un groupe benzyle.

Par le terme « groupe alcoxy », on entend au sens de la présente invention tout groupe alcoxy de 1 à 6 atomes de carbones, linéaire ou ramifié, en particulier, les groupe OCH₃ et OC₂H₅.

Par le terme « groupe alcoxycarbonyle », on entend au sens de la présente invention tout groupe alcoxy tel que défini ci-dessus lié par l'intermédiaire d'un groupe carbonyle. Un exemple de groupe alcoxycarbonyle est le groupe acétyle. Avantageusement, il s'agit d'un groupe tert-butyloxycarbonyle.

Par le terme « groupe aryloxy », on entend au sens de la présente invention tout groupe aryle tel que défini ci-dessus, lié par l'intermédiaire d'un atome d'oxygène. Avantageusement, il s'agit du groupe benzyloxy.

Par le terme « groupe aryloxycarbonyle », on entend au sens de la présente invention tout groupe aryloxy tel que défini ci-dessus, lié par l'intermédiaire d'un groupe carbonyle. Avantageusement, le groupe aryloxycarbonyle est le groupe carbobenzyloxy.

Par le terme « alkoxycarbonylalkyle », on entend au sens de la présente invention tout groupe alcoxy tel que défini ci-dessus, lié par l'intermédiaire d'un carbonyle lui-même lié par l'intermédiaire d'un groupe alkyle tel que défini ci-dessus. Avantageusement, il s'agit d'un groupe CH₂COOC₂H₅ ou CH₂CH₂COOCH₃.

Par le terme « groupe N-protecteur », on entend au sens de la présente invention tout substituant qui protège le groupe NH₂ contre les réactions indésirables tels que les groupes N-protecteur décrits dans Greene, "Protective Groups In Organic Synthesis", (John Wiley & Sons, New York (1981)) et Harrison et al. « Compendium of Synthetic Organic Methods", Vols. 1 à 8 (J. Wiley & sons, 1971 à 1996). Les groupes N-protecteur comprennent les carbamates, amides, dérivés N-alkylés, dérivés amino acétal, dérivés N-benzylé, dérivés imine, dérivés énamine et dérivés N-hétéroatome. En particulier, le groupe N-protecteur comprend le formyle, l'acétyle, le benzoyle, le pivaloyle, le phénylsulfonyle, le benzyle (Bn), le t-butyloxycarbonyle (boc), le benzyloxycarbonyle (cbz), le p-méthoxybenzyloxycarbonyle, le p-nitrobenzyloxycarbonyle, le trichloroéthoxycarbonyl (troc), l'allyloxycarbonyl (alloc), le 9-fluorénylméthyloxycarbonyl (Fmoc), le trifluoro-acétyle, les carbamates de benzyle (substitués ou non) et similaires. Il est avantageux d'utiliser soit du boc soit du cbz en tant que groupe N-protecteur en raison de la facilité relative d'élimination, par exemple par des acides modérés dans le cas du boc, par exemple l'acide trifluoroacétique ou l'acide chlorhydrique dans de l'acétate d'éthyle ; ou par une hydrogénation catalytique dans le cas de cbz. Avantageusement, il s'agit du groupe boc.

Par le terme « atome d'halogène » on entend au sens de la présente invention tout atome d'halogène, avantageusement choisi parmi Cl, Br, I ou F.

Dans la présente invention, on entend désigner par « pharmaceutiquement acceptable » ce qui est utile dans la préparation d'une composition pharmaceutique qui est généralement sûr, non toxique et ni biologiquement ni autrement non souhaitable et qui est acceptable pour une utilisation vétérinaire de même que pharmaceutique humaine.

On entend désigner par « sels pharmaceutiquement acceptables » d'un composé des sels qui sont pharmaceutiquement acceptables, comme défini ici, et qui possèdent l'activité pharmacologique souhaitée du composé parent. De tels sels comprennent :
(1) les sels d'addition d'acide formés avec des acides inorganiques tels que l'acide chlorhydrique, l'acide bromhydrique, l'acide sulfurique, l'acide nitrique, l'acide phosphorique et similaires ; ou formés avec des acides organiques tels que l'acide acétique, l'acide benzènesulfonique, l'acide benzoïque, l'acide camphresulfonique, l'acide citrique, l'acide éthane-sulfonique, l'acide fumarique, l'acide glucoheptonique, l'acide gluconique, l'acide glutamique, l'acide glycolique, l'acide hydroxynaphtoïque, l'acide 2-hydroxyéthanesulfonique, l'acide lactique, l'acide maléique, l'acide malique, l'acide mandélique, l'acide méthanesulfonique, l'acide muconique, l'acide 2-naphtalènesulfonique, l'acide propionique, l'acide salicylique, l'acide succinique, l'acide dibenzoyl-L-tartrique, l'acide tartrique, l'acide p-toluènesulfonique, l'acide triméthylacétique, l'acide trifluoroacétique et similaires ; ou
(2) les sels formés lorsqu'un proton acide présent dans le composé parent soit est remplacé par un ion métallique, par exemple un ion de métal alcalin, un ion de métal alcalino-terreux ou un ion d'aluminium ; soit se coordonne avec une base organique ou inorganique. Les bases organiques acceptables comprennent la diéthanolamine, l'éthanolamine, N-méthylglucamine, la triéthanolamine, la trométhamine et similaires. Les bases inorganiques acceptables comprennent l'hydroxyde d'aluminium, l'hydroxyde de calcium, l'hydroxyde de potassium, le carbonate de sodium et l'hydroxyde de sodium.

Les sels pharmaceutiquement acceptables préférés sont les sels formés à partir d'acide chlorhydrique ou d'acide trifluoroacétique.

Les composés selon l'invention possèdent tous un centre d'asymétrie et peuvent donc exister sous forme d'isomères optiques. La présente invention comprend aussi bien ces isomères soit séparément soit en tant que mélange.

Toutefois, avantageusement le procédé selon la présente invention est totalement énantiocontrôlé et les composés obtenus sont optiquement purs.

Avantageusement, dans le cas où Y représente le groupe ortho-phénylène, l'étape (a₁) consiste à faire réagir avec une base le composé de formule (VI) suivante : 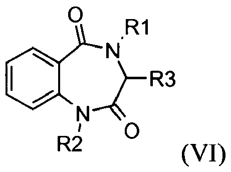 dans laquelle R1, R2 et R3 sont tels que définis précédemment.

R1 et R2 peuvent être avantageusement choisis indépendamment l'un de l'autre dans le groupe comprenant les alcoxycarbonyles en C₁ à C₆, les aryloxycarbonyles et le benzoyle, de préférence le tert-butyloxycarbonyle ou le carbobenzyloxy.

R1 et R2 sont avantageusement le tert-butyloxycarbonyle.

R3 et R4 peuvent être avantageusement choisis indépendamment l'un de l'autre dans le groupe comprenant l'isopropyle, le benzyle, le méthyle, le *sec-*butyle, le prényle (ou 3,3-diméthylallyle), l'allyle, -CH₂COOC₂H₅ et - CH₂CH₂COOCH₃.

La base peut être choisie dans le groupe comprenant le tert-butylate de potassium, l'hydrure de sodium, la lithium diisopropylamine, le lithium hexaméthyldisilazane, le potassium hexaméthyldisilazane et le diméthylaminopyridine.

La base est avantageusement le *tert*-butylate de potassium ou le lithium hexaméthyldisilazane ou l'hydrure de sodium. Avantageusement dans le cas de l'étape (a1) la base est le *tert*-butylate de potassium. Dans ce cas, l'étape (a1) peut comprendre, suite à la réaction avec la base, une étape de lavage avec HCl. Avantageusement dans le cas des étapes (a2), (a3) ou (a4), la base est le lithium hexaméthyldisilazane ou l'hydrure de sodium.

Le solvant de réaction est un solvant organique, avantageusement le tetrahydrofurane (THF).

Avantageusement, la réaction a lieu a température ambiante. Toutefois elle peut également avoir lieu à une température inférieure, avantageusement à une température comprise entre -78°C et la température ambiante, de façon avantageuse à 0°C ou à -78°C. En particulier la température de réaction dépend de la base utilisée. Ainsi avec NaH, la température est avantageusement de 0°C, avec LIHMDS, la température est avantageusement de -78°C et avec tBuOK, la réaction a avantageusement lieu à température ambiante.

Dans le cas où Y représente un groupe -C(HR4)- et R3 et R4 sont identiques et représentent un groupe allyle, le procédé comprend une étape supplémentaire (b₁) consistant à faire subir au composé de formule (I) une réaction de métathèse des oléfines intramoléculaire pour donner le composé de formule (VII) suivante : 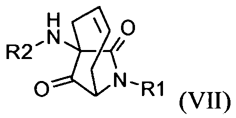 dans laquelle R1 et R2 sont tels que définis précédemment.

Dans le cas où Y représente le groupe -C(HR4)-, le procédé comprend les étapes successives supplémentaires suivantes consistant à :
- (b₂) faire réagir le composé de formule (I) en milieu basique avec un halogénure d'allyle pour donner le composé de formule (VIII) suivante : 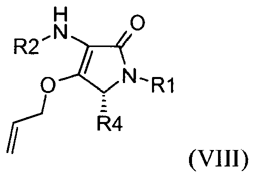 dans laquelle R1, R2 et R4 sont tels que définis précédemment, de préférence R4 est l'isopropyle,
- (c) convertir par chauffage, de préférence aux micro-ondes, le composé de formule (VIII) en composé de formule (IX) suivante : 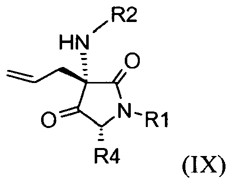 dans laquelle R1, R2 et R4 sont tels que définis précédemment, de préférence R4 est l'isopropyle,
- (d) faire réagir le composé de formule (IX) avec un halogénure d'allyle pour former le composé de formule (X) suivante : 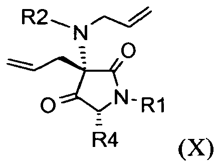 dans laquelle R1, R2 et R4 sont tels que définis précédemment, de préférence R4 est l'isopropyle, et
- (e) convertir le composé de formule (X) par métathèse des oléfines intramoléculaire en composé de formule (XI) suivante : 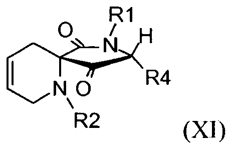
dans laquelle R1, R2 et R4 sont tels que définis précédemment, de préférence R4 est l'isopropyle. Avantageusement cette métathèse cyclisante est réalisée selon Grubbs. De façon avantageuse les étapes (c) et (d) sont un réarrangement type Claisen.

Dans le cas où R3 représente le groupe EtOOCCH₂- et Y représente le groupe -C(HR4)-, le procédé comprend une étape supplémentaire (b₃) consistant à faire réagir le composé de formule (I) avec un hydrure, avantageusement un borohydrure, de façon avantageuse NaBH₄, pour former le composé de formule (XII) suivante : 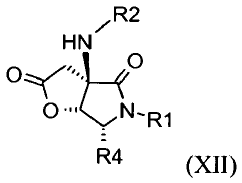 dans laquelle R1, R2 et R4 sont tels que définis précédemment, de préférence R4 est l'isopropyle.

Lorsque Y représente un groupe -(CHR4)-, le procédé comprend une étape supplémentaire (b₄) consistant à faire réagir le composé de formule (I) avec l'acide trifluoroacétique, avantageusement à une concentration massique comprise entre 3% et 10%, de préférence égale à 5% pour donner le composé de formule (XIII) suivante : 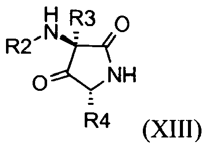 dans laquelle R2, R3 et R4 sont tels que définis précédemment, de préférence R4 est l'isopropyle.

Lorsque Y représente un groupe -(CHR4)-, le procédé comprend une étape supplémentaire (c₄) consistant à faire réagir le composé de formule (XIII) avec un hydrure, de préférence un borohydrure et plus préférentiellement NaBH₄, pour former le composé de formule (XIV) suivante : 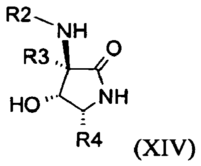 dans laquelle R2, R3 et R4 sont tels que définis précédemment, de préférence R4 est l'isopropyle.

Lorsque Y représente un groupe -(CHR4)-, le procédé comprend une étape supplémentaire (c₅) consistant à faire réagir le composé de formule (XIII) avec l'acide trifluoroacétique, avantageusement à une concentration massique comprise entre 10% et 25%, de préférence égale à 15% pour donner le composé de formule (XV) suivante : 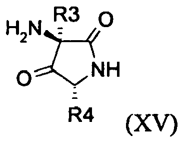 dans laquelle R3 et R4 sont tels que définis précédemment, de préférence R4 est l'isopropyle.

Dans le cas où Y représente le groupe -(CHR4)-, le procédé comprend une étape supplémentaire (b₅) consistant à faire réagir le composé de formule (I) avec un borohydrure, de préférence NaBH₄, pour former le composé de formule (XVI) suivante : 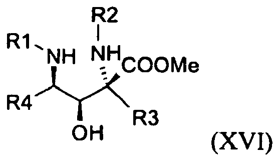 dans laquelle R1, R2, R3 et R4 sont tels que définis précédemment.

Les procédés de la présente invention ouvrent une voie supplémentaire vers plusieurs familles de molécules d'intérêt biologique ou pharmaceutique important, ayant fait l'objet de nombreuses recherches et publications. Ces procédés donnent en effet accès à une variété d'intermédiaires clés dans la synthèse totale de molécules biologiquement actives, ou permettent de synthétiser des composés possédant des propriétés intéressantes.

Ainsi, les composés de formule (VIII) sont structurellement comparables à la vitamine C, bien connue entre autres pour ses propriétés antioxydantes.

Les composés de formule (XVI) possèdent la sous-structure peptidique de la bleomicine (antibiotique et anti-cancéreux), de la janolusimide et des statines (inhibiteurs enzymatiques).

Les composés de formule (XII) sont analogues à la lactone de l'isocitrimide de la mescaline, une substance psycho-active issue du mescal.

Les composés de formule (VI) sont apparentés à des composés présentant des propriétés anti-tumorales, anti-VIH, antihypertenseur, anti-inflammatoires, analgésiques, et ont un pouvoir relaxant sur les muscles.

L'invention va maintenant être illustrée, de façon non limitative, par les exemples suivants.

### Exemple 1 : synthèse de la 1-tert-butoxycarbonyl-4-hydroxy-3-tert-butyloxycarbonylamino-1,5-dihydropyrrol-2-one (I-a).

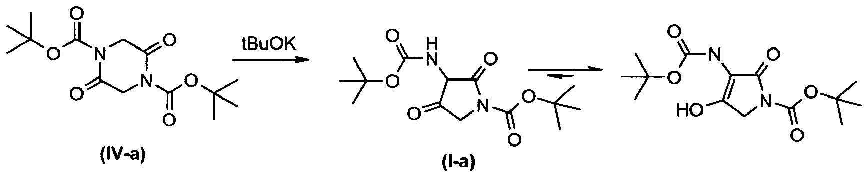

A une solution de la pipérazine-2,5-dione **(IV-a)** substituée (0,794 g, 2,53 mmol) dans du THF déshydraté (10,3 mL), on a ajouté du t-BuOK (0,312 g, 2,78 mmol) à température ambiante. La solution a été agitée pendant 12 heures sous une atmosphère d'argon. Le milieu a ensuite été dilué au moyen d'AcOEt (20 mL), lavé avec une solution de HCl 0,1N et séché sur du MgSO₄ anhydre. Le solvant a été éliminé *in vacuo* et le résidu brut a été purifié par chromatographie sur colonne de gel de silice (dichlorométhane/MeOH 100/0→90/10). Le lactame **(I-a)** a été isolé sous la forme d'un solide incolore (0,575g) correspondant à la forme énolique.
Rendement = 72 % (composé pur). Point de fusion = 65°C, non corrigé.
¹H-RMN (CDCl₃, 200 MHz) : δ 1,51 (s, 9H, CH₃C) ; 1,55 (s, 9H, CH₃C) ; 4,16 (s, 2H, CH₂) ; 6,65 (s1, 1H, NH) ; (DMSO-d6, 200 MHz) : δ 1,40 (s, 9H, CH₃C) ; 1,46 (s, 9H, CH₃C) ; 4,12 (s, 2H, CH₂) ; 7,84 (s1, 1H, NH) ; 11,96 (m, 1H, OH). ¹³C-RMN (CDCl₃, 100 MHz) : δ 28,0 (CH₃C) ; 47,0 (CH₂); 83,1 et 83,3 (CCH₃); 104,1 (CvinN); 148,9 et 155,7 (CO carbamate) ; 150,8 (CvinO); 165,1 (CO lactame). LC/MS : tr = 3,84 min ; 646, [2M+NH4⁺] ; 629 ; [2M+H⁺] ; 332, [M+NH4⁺] ; 315, [M+H⁺] ; 259, [(M+H⁺) - tBu] ; 203, [(M+H⁺) - 2tBu] ; 159 [(M+H⁺) - 2Boc]. tr = 2,66 min 2 avec la perte d'un group Boc in situ : 215 [(M+H⁺-Boc)]. HPLC = 11,46 min.

### Exemple 2 : synthèse des (3S,5S) 1-tert-butoxycarbonyl-3-tert-butoxycarbonylamino-3,5-diméthylpyrrolidine-2,4-dione (I-b1) et (3R,5S) (I-b2)

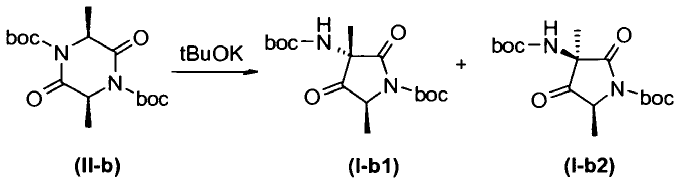

Les composés ont été synthétisés selon la procédure décrite plus haut. **(I-b1)** (3S,5S) ; point de fusion = 104°C (non corrigé) ; [α]D20 = -9,0 (c = 2,12, dichlorométhane). ¹H-RMN (CDCl₃, 200 MHz) : δ 1,35 (s, 9H, CH₃C) ; 1,40 (d, 3H, CH₃) ; 1,52 (s, 3H, CH₃ quat) ; 1,54 (s, 9H, CH₃C) ; 4,52 (q, 1H, CH*, J = 7,1 Hz) ; 5,54 (sb, 1H, NH). nOe entre CHαAla et NH Boc. ¹³C-RMN (CDCl₃, 100 MHz) : δ 17,9, 20,2 (CH₃ Ala); 28,0, 28,1 (CH₃C Boc); 59,5 (Cquat); 60,6 (CH*) ; 81,5, 84,0 (CCH₃) ; 149,0, 154,9 (CO carbamate) ; 172,0 (CO lactame) ; 207,1 (CO cétone). LC/MS : tr = 3,75 min ; 343 [M+H⁺]; 287 [(M+H⁺) - tBu] ; 231 [(M+H⁺) - 2tBu]. HR-MS 343,1870 Th (calculé = 343,1869 Th).
HPLC; tr = 11,153 min.
**(I-b1)** (3R,5S) ; point de fusion = 159°C (non corrigé) ; [α]D20 = -101,3 (c = 1,57, dichlorométhane). ¹H-RMN (CDCl₃, 200 MHz): δ 1,41 (s, 9H, CH₃C); 1,43 (s, 3H, CH₃Cquat) ; 1,59 (s, 9H, CH₃C) ; 1,69 (d, 3H, CH₃CH*, J = 6,6 Hz) ; 4,47 (q, 1H, CH*, J = 6,7 Hz); 5,23 (sb, 1H, NH), NOe entre CHα Ala et CH₃Cquat, et entre CH₃CH* et NH-Boc. ¹³C-RMN (CDCl₃, 100 MHz) : δ 16,4 (CH₃CH*) ; 19,8 (CH₃Cquat) ; 28,0, 28,1 (CH₃C) ; 59,9 (CH*) ; 60,0 (Cquat), 81,4, 84,0 (CCH₃); 149,1 , 154,4 (CO carbamate) ; 171,6 (CO lactame) ; 207,2 (CO cétone). LC/MS : tr = 3,90 min : 343 [M+H⁺] ; 287 [(M+H⁺) - tBu] ; 231 [(M+H⁺) - 2tBu] ; 143 [(M+H⁺) - 2Boc]. HR-MS 343,1870 Th (calculé = 343,1869 Th). HPLC tr = 11,602 min.

### Exemple 3 : synthèse des 1-tert-butoxycarbonyl-3-benzyl-3-tert-butoxycarbonylaminopyrrolidine-2,4-dione (I-c), 1-tert-butyloxycarbonyl-3-tert-butoxycarbonylamino-3,5-dibenzylpyrrolidine-2,4-dione (I-d), et 3-tert-butyloxycarbonylamino-3,5-diallyl-1-tert-butoxycarbonylpyrrolidine-2,4-dione (I-e)

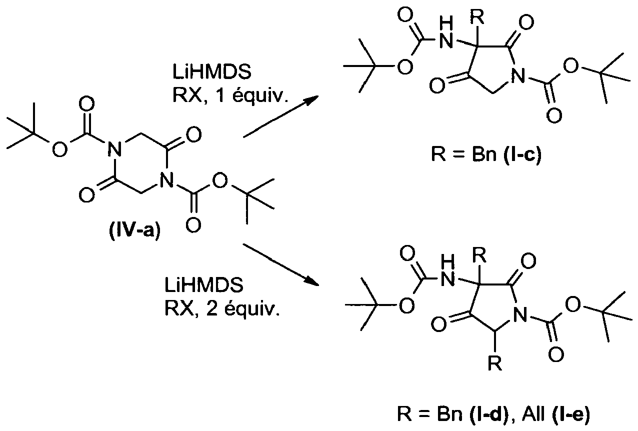

A une solution agitée du composé (IV-a) (0,237 g, 0,75 mmol) dans du THF déshydraté (3,8 mL), on a ajouté goutte à goutte une solution de LiHMDS 1,06 M dans du THF (1,78 mL, 1,89 mmol) à -78°C sous une atmosphère d'argon. Au bout de 45 min, on a ajouté du bromure de benzyle (0,20 mL, 1,66 mmol). Le mélange réactionnel a été laissé à température ambiante pendant 12 heures, puis dilué avec de l'AcOEt (10 mL) et lavé au moyen d'une solution de HCl 0,1N. La phase organique a été séchée sur du MgSO₄ et le solvant a été concentré *in vacuo.* Le mélange brut de composés alkylés a été purifié par chromatographie sur colonne de gel de silice (dichlorométhane-AcOEt), ce qui a donné successivement les composés **(I-d)** (0,092 g) et **(I-c)** (0,058 g), sous la forme de poudres incolores.
**(I-c)** : point de fusion 126°C (non corrigé). ¹H-RMN (DMSO-d6, 200 MHz) : δ 1,36 (s, 9H, CH3C) ; 1,40 (s, 9H, CH3C) ; 2,93, 3,90 (dd, 2H, CH₂ Gly, JAB = 18,4 Hz) ; 2,96, 3,05 (dd, 2H, CH₂Ph, JAB = 12,6 Hz) ; 7,00 - 7,35 (m, 5H, Harom) ; 8,42 (s, 1H, NH), nOe détecté entre le proton le plus protégé du Gly méthylène (proS) et les protons aromatiques. 13C-RMN (CDCl₃, 100 MHz) : δ 27,9, 28,2 (CH₃C) ; 40,5 (CH₂Ph) ; 54,6 (CH₂ Gly) ; 65,2 (CCH₂Ph) ; 81,9, 83,7 (CCH₃); 126,6 -135,7 (Carom) ; 148,3, 154,8 (CO carbamates); 171,0 (CO lactame). HPLC : tr = 12,779 min. LC/MS : tr = 4,40 min, 809 [2M+H⁺] ; 405 [M+H⁺] ; 349 [(M+H⁺) - tBu] ; 305 [(M+H⁺) - Boc] ; 249 [(M+H⁺) - Boc - tBu] ; 205 [(M+H⁺) - 2Boc]. HR-MS : 405,2048 Th (calculé = 405,2026 Th) ; C21H28N2O6.
(I-d) : point de fusion 147°C (non corrigé). ¹H-RMN (DMSO-d6, 200 MHz) : δ 1,32 (s, 9H, CH₃C) ; 1,45 (s, 9H, CH₃C) ; 1,65, 2,30 (ddd, 2H, CH* CH₂Ph, JAB = 13,9 Hz, JAX = 8,8 Hz, JBX = 4,3 Hz) ; 2,62, 2,97 (dd, 2H, Cquat CH₂Ph, JA'B' = 13,3 Hz) ; 4,3, 4,44 (dd, 1H, JAX = 8,7 Hz, JBX = 4,3 Hz) ; 6,98- 7,44 (m, 5H, Harom); 8,31 (s, 1H, NH). ¹³C-RMN (CDCl₃, 100 MHz) : δ 28,1 (CH₃C) ; 34,9 (CH*CH₂Ph) ; 37,7 (CquatCH₂Ph) ; 61,8 (CquatCH₂Ph) ; 66,1 (CH*CH₂Ph) ; 81,6, 84,3 (CCH₃) ; 128,3, 130,8 (Carom); 149,3, 154,9 (CO carbamate) ; 171,4 (CO lactame) ; 205,4 (CO cétone). HPLCtr = 14,50 min.

LC/MS : tr = 4,99 min : 495 [M+H⁺] ; 439 [(M+H⁺) - tBu] ; 395 [(M+H⁺) - Boc] ; 339 [(M+H⁺) - Boc - tBu] ; 295 [(M+H⁺) - 2Boc]. HR-MS : 495,2528 Th (calculé = 495,2495 Th) C28H34N2O6.
**(I-e):** préparé par analogie avec du bromure d'allyle. Huile incolore (mélange d'isomères géométriques).
¹H-RMN (CDCl₃, 200 MHz) : δ 1,37 (s, 9H, CH₃C) ; 1,39 (s, 9H, CH₃C) ; 1,58 (s, 9H, CH₃C) ; 1,64 (s, 9H, CH₃C) ; 2,31 - 2,61 (m, 4H, CquatCH₂CH=CH₂) ; 2,61 - 3,05 (m, 4H, CH* CH₂CH=CH₂) ; 4,22 - 4,31 (m, 1H, CH*) ; 4,56 - 4,66 (m, 1H, CH*) ; 5,04 - 5,35 (m, 10H, NH et CH₂=CH) ; 5,53 - 6,14 (m, 4H, CH=CH₂). LC/MS tr = 4,38 min : 395 [M+H⁺] ; 339 [(M+H⁺) - tBu] ; 295 [(M+H⁺) - Boc] ; 283 [(M+H⁺) - 2 tBu].

### Exemple 4 : synthèse de l'ester tert-butylique de l'acide 7-tert-butoxycarbonyl-8,9-dioxo-7-aza-bicyclo[4.2.1]non-3-en-1-yl-carbamique (VII-e)

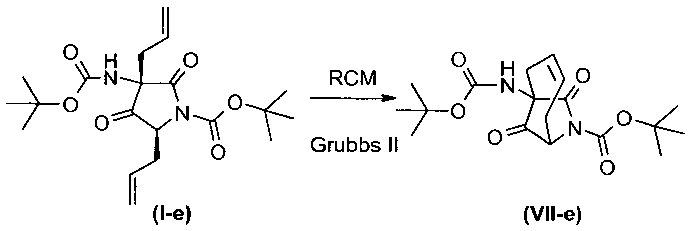

A une solution portée à reflux du bis-allyltétramate **(I-e)** (0,226 g, 0,57 mmol) dans du dichlorométhane fraîchement distillé (8 mL), on a ajouté du benzylidène-[1,3-bis-(2,4,6-triméthylphényl-2-imidazo lidinylidène]-dichloro(tricyclohexylphosphane) ruthénium (réactif de Grubbs II) (0,010 g, 0,0115 mmol). La solution a été agitée pendant 30 min à 45°C et désactivée par du DMSO (0,04 mL, 0,58 mmol, 50 equiv. par rapport au catalyseur). Après 12 heures d'agitation à température ambiante, le solvant a été concentré *in vacuo* et le résidu brut purifié par chromatographie sur colonne de gel de silice (dichlorométhane/AcOEt (100/0→85/15). Le composé bicyclique **(VII-e)** a été récupéré sous la forme d'un solide blanc (0,144 g). Rendement 70 %.
Point de fusion : 104°C (non corrigé).
¹H-RMN (CDCl₃, 200 MHz) : δ 1,35 (s, 6H, CH₃C) ; 1,42 (s, 3H, CH₃C) ; 1,54 (s, 9H, CH₃C) ; 2,22- 2,33 et 2,55 - 2,68 (m, 2H, CH₂Cquat) ; 2,36 - 2,48 et 2,80 - 2,92 (m, 2H, CH₂CH*) ; 4,58 (m, 0,3H, CH*) ; 4,70 (m, 0,7H, CH*) ; 5,40 (s, 1H, NH) ; 5,46 à 5,61 (m, 2H, CH=CH). L'isomérisme E/Z sur le groupe Boc-lactame a probablement été responsable du dédoublement du signal lié à tBu et du signal du CH* voisin. ¹³C-RMN (CDCl₃, 100 MHz) : δ 28,0 et 28,2 (CH₃C) ; 30,3 , 33,7 (CH₂) ; 62,0 (CH*) ; 64,4 (Cquat) ; 81,6, 84,2 (CCH₃) ; 121,9, 124,5 (CH=CH) ; 148,4, 154,6 (CO carbamate) ; 170,3 (CO lactame) ; 204,7 (CO cétone). HPLCtr = 10,937 min LC/MS : tr = 3,89 min : 367 [M+H⁺] ; 311 [(M+H⁺) - tBu] 267 [(M+H⁺) - Boc]. C18H26N2O6.

### Exemple 5 : synthèse des (3R,5R) 1-tert-butoxycarbonyl-3-tert-butoxycarbonylamino-5-isopropyl-3-méthylpyrrolidine-2,4-dione (I-f), (3R,5R) 1-tert-butoxycarbonyl-3-tert-butoxycarbonylamino-5-isopropyl-3-benzylpyrrolidine-2,4-dione (I-g) et (3R,5R) 1-tert-butoxycarbonyl -3-tert-butoxycarbonylamino-5-isopropyl-3-allylpyrrolidine-2,4-dione (I-h)

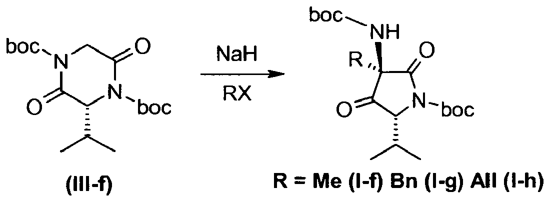

(3R, 5R) 1-*tert*-butoxycarbonyl-3-*tert*-butoxycarbonylamino-5-*isopropyl*-3-méthylpyrrolidine-2,4-dione **(I-f)**
Une suspension de NaH 60 % dans de l'huile minérale (0,076 g, 3,17 mmol) a été diluée dans du THF déshydraté (60 mL). Une solution de la pipérazinedione 3 (0,753 g, 2,11 mmol), dissoute dans du THF déshydraté, a été ajoutée goutte à goutte à 0°C sous une atmosphère d'argon. La réaction a été agitée pendant 1 heure et de l'iodure de méthyle (0,16 mL, 2,54 mmol) a été ajouté. Après 12 heures d'agitation, on a ajouté de l'AcOEt (24 mL). La phase organique a été lavée avec du HCl 0,1N, séchée sur du MgSO₄ et concentrée *in vacuo.* Le résidu brut a ensuite été purifié par chromatographie sur colonne de gel de silice (éther de pétrole/AcOEt 100/0→50/50). Le composé **(I-f)** a été obtenu sous la forme d'une poudre blanche (0,450 g, rendement 60 %).
Point de fusion : 111°C (non corrigé). [α]D20 = +11 (c = 2,10, dichlorométhane) ¹H-RMN (CDCl₃, 200 MHz) : δ 0,93 (d, 3H, CH₃CH, J = 7,0 Hz) ; 1,20 (d, 3H, CH₃CH, J = 7,1 Hz) ; 1,36 (s, 9H, CH₃C) ; 1,38 (s, 3H, CH₃Cquat) ; 1,56 (s, 9H, CH₃C) ; 2,32 -2,50 (m, 1H, CH CH₃) ; 4,53 (d, 1H, CH*, J = 4,2 Hz) ; 5,36 (sb, 1H, NH). ¹³C-RMN (CDCl₃, 100 MHz) : δ 16,5, 17,8 (CH₃CH) ; 18,7 (CH₃Cquat) ; 28,0 (CH₃C) ; 29,7 (CH) ; 59,3 (Cquat); 69,9 (CH*); 81,3, 83,8 (CCH₃) ; 149,6, 154,7 (CO carbamates) ; 172,4 (CO lactame) ; 206,7 (CO cétone). HPLC ; tr = 12,454 min. LC/MS : tr = 4,22 min : 371 [M+H⁺] ; 315 [(M+H⁺) - tBu] ; 271 [(M+H⁺) - Boc] ; 259 [(M+H⁺) - 2 tBu] ; 215, [(M+H⁺) - Boc - tBu] ; 171 [(M+H⁺) - 2 Boc]. HR-MS : 371,2167 Th (calculé = 371,2182 Th) pour C18H30N2O6.

Les composés **(I-g)** et **(I-h)** ont été synthétisés à partir du composé **(III-f)** par la même procédure et les mêmes préparatifs, en utilisant respectivement, comme agents alkylants, le bromure de benzyle et le bromure d'allyle.
(3R,5R) 1-*tert*-butoxycarbonyl-3-*tert*-butoxycarbonylamino-5-isopropyl-3-benzylpyrrolidine-2,4-dione **(I-g)**
Rendement 42 %. Cristaux incolores. Point de fusion : 114°C (non corrigé).
[α]D20 = + 20 (c = 2,01, dichlorométhane). Dichroïsme circulaire : trifluoroéthanol (λnm, θ) : 193, 47 500 ; 205, 0 ; 225, -18000 ; 250, - 11000. ¹H-RMN (CDCl₃, 200 MHz) : δ 1,01 (d, 3H, CH₃CH, J = 6,8 Hz) ; 1,08 (d, 3H, CH₃CH, J = 7,0 Hz) ; 1,32 (s, 9H, CH₃C) ; 1,56 (s, 9H, CH₃C) ; 1,92 - 2,16 (m, 1H, CHCH₃) ; 3,03 (sb, 2H, CH₂) ; 4,47 (d, 1H, CH*, J = 5,6 Hz) ; 5,11 (sb, 1H, NH) ; 7,15 - 7,40 (m, 5H, Harom). ¹³C-RMN (CDCl₃, 100 MHz) : δ 18,7, 19,3 (CH₃CH) ; 28,0 (CH₃C) ; 30,1 (CH) ; 38,0 (CH₂) ; 61,8 (CCH₂Ph) ; 69,7 (CH*) ; 81,4, 84,0 (CH3) ; 128,2 -131,8 (Carom) ; 149,8, 154,8 (CO carbamate) ; 171,7 (CO lactame) ; 206,1 (CO cétone). HPLCtr = 14,324 min ; LC/MS : tr = 4,83 min : 447 [M+H⁺] ; 391 [(M+H⁺)-tBu] ; 347 [(M+H⁺) - Boc] ; 291 [(M+H⁺) - Boc - tBu]. HR-MS 447,2521 Th (calculé = 447,2495 Th) C24H34N206.
(3R,5R) 1-*tert*-butoxycarbonyl-3-*tert*-butoxycarbonylamino-5-isopropyl-3-allylpyrrolidine-2,4-dione **(I-h)**
Rendement 42 %. Cristaux incolores. Point de fusion : 83°C (non corrigé). [α]D20 = + 15 (c= 1,92, dichlorométhane). Dichroïsme circulaire : trifluoroéthanol (λnm, θ) : 193, 33 000; 206, 0; 225, -11000; 250, -13000. ¹H-RMN (CDCl₃, 200 MHz) : δ 0,98 (d, 3H, CH₃CH, J = 7,0 Hz) ; 1,22 (d, 3H, CH₃CH, J = 7,1 Hz) ; 1,38 (s, 9H, CH₃C); 1,58 (s, 9H, CH₃C); 2,34 - 2,61 (m, 3H, CHCH₃ et CH₂CH=CH₂) 4,52 (d, 1H, CH*, J = 4,4 Hz); 5,21 - 5,37 (m, 3H, NH et CH₂=CH) ; 5,82 - 6,03 (m, 1H, CH=CH₂).

### Exemple 6 : synthèse de la (R) 4-allyloxy-3-tert-butoxycarbonylamino-5-isopropyl-1-tert-butoxycarbonyl-1,5-dihydropyrrol-2-one (VIII-i) et de la (3S,5R)-3-allyl-3-tert-butoxycarbonylamino-5-isopropyl-1-tert-butoxycarbonylpyrrolidine-2,4-dione (IX-i)

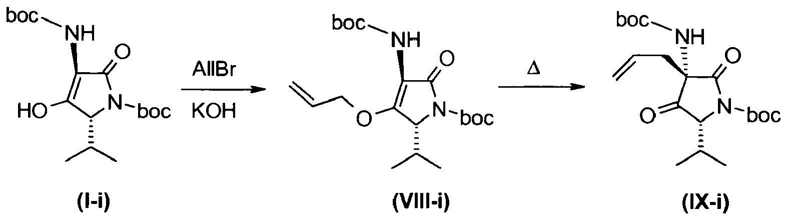

(R) 4-allyloxy-3-*tert*-butoxycarbonylamino-5-isopropyl-1-*tert*-butoxycarbonyl-1,5-dihydropyrrol-2-one **(VIII-i)**
A une solution du composé **(I-i)** (0,521 g, 1,46 mmol) dans du DMSO déshydraté (5,5 mL), on a ajouté du KOH en poudre (0,074 g, 1,32 mmol) sous agitation. Un léger chauffage s'est révélé nécessaire pour une dissolution complète. Le mélange s'est coloré, passant du rose au violet foncé. Du bromure d'allyle (0,11 mL, 1,32 mmol) a alors été ajouté et le mélange réactionnel a été agité pendant 6 heures sous une atmosphère d'argon. De l'AcOEt (11 mL) a alors été ajouté. La phase organique a été lavée avec du HCl 0,1N, séchée sur du sulfate de magnésium anhydre et concentrée *in vacuo.* Le résidu brut a été purifié par chromatographie sur colonne de gel de silice (dichlorométhane/AcOEt 100/0→80/20), ce qui a donné le dérivé allyloxy **(VIII-i)** (0,365 g) sous la forme d'une poudre blanche avec un rendement de 60 %.
[α]D20 = - 23 (c = 0,24, MeOH). ¹H-RMN (CDCl₃, 200 MHz) : δ 0,90 (d, 3H, CH₃CH, J = 7,0 Hz) ; 1,14 (d, 3H, CH₃CH, J = 7,2 Hz) ; 1,48 (s, 9H, CH₃C) ; 1,56 (s, 9H, CH₃C) ; 2,45-2,53 (m, 1H, CHCH₃) ; 4,20 (d, 1H, CH*, J = 3,2 Hz) ; 4,90 (m, 2H, CH₂O): 5,29 (d, 2H, CH₂=CH, J = 1,4 Hz) ; 5,65 (sb, 1H, NH) ; 5,93-6,02 (m, 1H, CH=CH₂). ¹³C-RMN (CDCl₃, 100 MHz) : δ 15,9, 18,6 (CH₃CH) ; 28,1 (CH₃C) ; 29,8 (CH CH₃) ; 62,6 (CH*) ; 71,3 (CH₂O) ; 81,1, 82,8 (CCH₃) ; 104,7 (CvinN); 118,9 (CH₂=CH); 132,1 (CH=CH₂); 149,2, 155,2 (CO carbamates) ; 165,5, 167,6 (CvinO and CO lactame). HPLC ; tr = 13,220 min ; LC/MS : tr = 3,06 min : [M+Na⁺] = 419 ; [M+H⁺] = 397 ; [(M+H⁺) - tBu] = 341 ; [(M+H⁺) - 2 tBu] = 285 ; [(M+H⁺) - Boc - tBu] = 241. HR-MS 397,2326 Th (calculé = 397,2339 Th).
(3S,5R)-3-allyl-3-*tert*-butoxycarbonylamino-5-isopropyl-1*-tert-*butoxycarbonylpyrrolidine-2,4-dione **(IX-i)**
Une solution du composé **(VIII-i)** (0,180 g, 0,45 mmol) dans du trifluorotoluène (3 mL) a été irradiée pendant 30 min à 170°C (dispositif Biotage). Le milieu a ensuite été concentré *in vacuo* et le résidu brut a été purifié par chromatographie sur colonne de gel de silice (dichlorométhane-AcOEt), ce qui a donné, après concentration du solvant, le composé **(IX-i)** sous la forme de cristaux blancs (0,088 g, 50 % de rendement).
[α]D20 = - 88 (c : 1,34, dichlorométhane). ¹H-RMN (CDCl₃, 200 MHz) : δ 1,13 (d, 3H, CH₃CH, J = 7,2 Hz) ; 1,17 (d, 3H, CH₃CH, J = 7,5 Hz) ; 1,39 (s, 9H, CH₃C); 1,58 (s, 9H, CH₃C) ; 2,47 (d, 2H, CH₂CH=CH₂, J = 7,5 Hz) ; 2,48 à 2,59 (m, 1H, CHCH₃) ; 4,21 (d, 1H, CH*, J = 4,9 Hz) ; 5,04 (sb, 1H, NH) ; 5,23 - 5,33 (m, 2H, CH₂=CH) ; 5,59 - 5,80 (m, 1H, CH=CH₂). ¹³C-RMN (CDCl₃, 100 MHz) : δ 19,0 et 19,2 (CH₃CH); 28,0 et 28,1 (CH₃C); 31,0 (CH CH₃); 39,2 (CH₂CH=CH₂); 63,6 (Cquat); 68,1 (CH*) ; 81,1 , 84,0 (C CH₃); 122,6 (CH₂=CH); 128,3 (CH= CH₂); 149,8, 153,9 (CO carbamates); 170,5 (CO lactame) ; 205,9 (CO cétone). HPLC : tr = 13,411 min ; LC/MS : tr = 4,62 min : 397 [M+H⁺] ; 341 [(M+H⁺) - tBu] ; 297 [(M+H⁺) - Boc] ; 285 [(M+H⁺) - 2 tBu] ; 197 [(M+H⁺) - 2 Boc]. HR-MS. : 397,2318 Th (calculé = 397,2339 Th) pour C20H32N2O6.

### Exemple 7 : synthèse du dérivé spiro D-Val-Baikiain (XI-a)

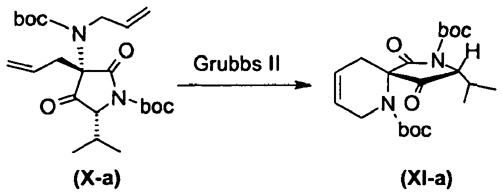

A une solution de diallylpyrrolidinedione **(X-a)** (0,105 g, 0,24 mmol) dans du dichlorométhane (5 mL) à reflux, on a ajouté du catalyseur de Grubbs (benzylidène-[1,3-bis-(2,4,6-triméthylphényl)-2-imidazolidinylidène]-dichloro (tricyclohexylphosphane) ruthénium (0,004 g, 0,0048 mmol, 2 % en mol) dans 2 mL du même solvant, sous agitation. Au bout de 2 heures, le milieu réactionnel a été désactivé au moyen de DMSO (0,02 mL, 0,24 mmol, 50 equiv. par rapport au catalyseur), puis le mélange a été agité pendant 12 heures et concentré *in vacuo.* Le mélange brut a été purifié par chromatographie sur colonne de gel de silice en utilisant du dichlorométhane comme éluant. Le composé **(XI-a)** (0,047 g, 48 % de rendement) a été isolé sous la forme de cristaux après élimination du solvant par concentration *in vacuo.*
Point de fusion = 124°C (non corrigé). [α]D20 = - 89 (c = 1,90, CH₂Cl₂). ¹H-RMN (CDCl₃, 300 MHz) : δ 1,04 (d, 3H, CH₃CH, J = 7,0 Hz) ; 1,08 (d, 3H, CH₃CH, J = 7,2 Hz) ; 1,35 (s, 9H, CH₃C) ; 1,50 (s, 9H, CH₃C) ; 2,24 - 2,36 (m, 1H, CH₂Cquat) ; 2,40 - 2,52 (m, 2H, CHCH₃ et CH₂Cquat) ; 3,98 - 4,02 (m, 2H, CH₂N) ; 4,23 (d, 1H, CH*, J = 4,5 Hz) ; 5,60 - 5,70 (m, 1H, CH=CHCH₂C) ; 5,86 - 5,94 (m, 1H, CH=CHCH₂N). ¹³C-RMN (CDCl₃, 75 MHz) : δ 19,1, 19,2 (CH₃CH) ; 28,0, 28,2 (CH₃C) ; 30,6 (CH₂Cquat) ; 30,9 (CHCH₃) ; 43,6 (CH₂N) ; 62,8 (Cquat); 67,0 (CH*); 81,7, 83,8 (CCH₃); 118,5 (CCH₂CH=CH); 126,0 (NCH₂CH=CH) ; 150,0, 155,1 (CO carbamate) ; 171,1 (CO lactame) ; 206,2 (CO cétone). HPLC. tr = 14,275 min ; LC/MS : tr = 3,23 min : 409 [M+H⁺] ; 353 [(M+H⁺) - tBu] ; 297 [(M+H⁺) - 2 tBu] ; 209 [(M+H⁺) - 2 Boc], HR-MS 409,2347 Th (calculé = 409,2339 Th) pour C21H32N2O6.

### Exemple comparatif 8 : synthèse des (3R,5R)1-tert-butoxycarbonyl-3-tert-butoxycarbonylamino-3-(éthoxycarbonylméthyl)-5-isopropyl-pyrrolidine-2,4-dione (1-j) et (3aR,6R,6aS) 3a-(tert-butoxycarbonyl)-6-isopropyl-2,4-dioxohexahydrofuro[2,3-c]pyrrole-5-carboxylate de tert-butyle (XII-j)

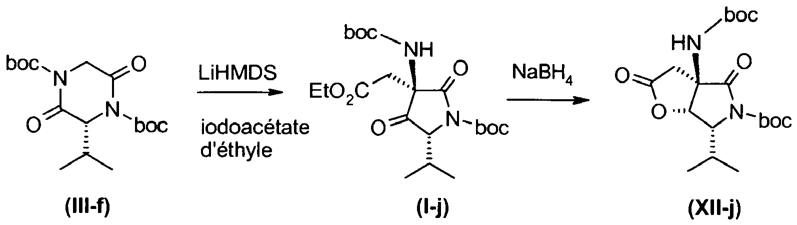

**(III-f)** a été synthétisé à partir de 3 et d'éthyliodoacétate en présence de LiHMDS selon la procédure décrite plus haut.
(3R,5R) 1-*tert*-butoxycarbonyl-3-tert-butoxycarbonylamino-3-(éthoxycarbonylméthyl)-5-isopropyl pyrrolidine-2,4-dione **(I-j)**
Huile jaune ; rendement 49 %. [α]D20 = + 21 (C = 1,31, CH₂Cl₂). Rf = 0,60 (hexane/AcOEt) (70/30). ¹H-RMN (CDCl₃, 300 MHz) : δ 0,98 (d, 3H, CH₃CH, J = 7,0 Hz) ; 1,20 (d, 3H, CH₃CH, J = 7,1 Hz) ; 1,29 (t, 3H, CH₃ CH₂, J = 7,0 Hz) ; 1,37 (s, 9H, CH₃C) ; 1,56 (s, 9H, CH₃C) ; 2,36 - 2,49 (m, 1H, CHCH₃) ; 2,49 - 2,62 (m, 2H, CH₂Cquat) ; 4,05 - 4,28 (m, 2H, CH₂CH₃); 4,54 (d, 1H, CH*, J = 4,3 Hz) ; 6,61 (s1, 1H, NH). ¹³C-RMN (CDCl₃, 75 MHz) : δ 14,0 (CH₃CH₂) ; 17,7, 19,1 (CH₃CH) ; 27,9, 28,1 (CH₃C) ; 30,1 (CH) ; 34,8 (CH₂Cquat) ; 59,8 (Cquat) ; 69,9 (CH*) ; 81,4, 84,3 (CCH3) ; 149,5, 155,0 (CO carbamate) ; 168,4 et 170,0 (CO lactame et CO ester) ; 204,6 (CO cétone). LC/MS : tr = 4,73 min : [M+H⁺] = 443 ; [(M+H⁺) - tBu] = 387 ; [(M+H⁺) - Boc] = 343 ; [(M+H⁺) - Boc - tBu] = 287 ; [(M+H⁺) - 2 Boc] = 243. HR-MS = 443,2411 Th (calculé 443,2393 Th) pour C21H34N2O8.
(3aR,6R,6aS) 3a-(*tert*-butoxycarbonyl)-6-isopropyl-2,4-dioxohexahydrofuro[2,3-c]pyrrole-5-carboxylate de *tert*-butyle (XII-j)
A une solution de (I-j) (0,040 g, 0,09 mmol) dans un mélange de THF/eau (4/1), on a ajouté du NaBH₄ (0,010 g, 0,27 mmol) à 0°C sous agitation. Au bout de 30 min, on a ajouté de l'AcOEt (10 mL). La phase organique a été lavée avec du HCl 0,1N, séchée sur du MgSO₄ et concentrée *in vacuo.* Le résidu brut a alors été purifié par chromatographie sur colonne de gel de silice (dichlorométhane/AcOEt 100/0→95/5). Le composé **(XII-j)** a été obtenu avec un rendement de 75 % (0,027 g) sous la forme d'une huile incolore.
[α]D20 = -23 (c = 1,11, dichlorométhane) ; Rf = 0,61 (hexane/AcOEt (60/40). ¹H-RMN (CDCl₃, 200 MHz) : δ 0,91 (d, 3H, CH₃CH, J = 7,0 Hz) ; 1,08 (d, 3H, CH₃CH, J = 7,1 Hz) ; 1,37 (s, 9H, CH₃C) ; 1,48 (s, 9H, CH₃C) ; 2,42 - 2,58 (m, 1H, CHCH₃) ; 2,85 (d, 1H, CH₂, JAB = 18,6 Hz) ; 2,94 (d, 1H, CH₂, JAB = 18,6 Hz) ; 4,29 (t, 1H, CH*CH(CH₃)₂, J = 6,3 Hz) ; 5,12 (s1, 1H, NHBoc) ; 5,20 - 5,26 (m, 1H, CH*O). ¹³C-RMN (CDCl₃, 100 MHz) : δ 17,1, 17,9 (CH₃CH) ; 27,8, 28,2 (CH₃C) ; 29,7 (CHCH₃) ; 36,7 (CH₂) ; 63,1 (CH*CH(CH₃)₂) ; 63,6 (CCH₂CO) ; 80,6 (CH*O) ; 81,8 et 84,7 (CCH₃); 149,8, 154,5 (CO carbame) ; 171,6 et 172,0 (CO lactame et lactone). HPLC : tr = 12,418 min; LC/MS : tr = 4,34 min :
[M+H⁺] = 399 ; [(M+H⁺) - tBu] = 343 ; [(M+H⁺) - 2 tBu] = 287 ; [(M+H⁺) - Boc - tBu] = 243. HR-MS 399,2143 Th (calculé = 399,2131 Th) pour C19H30N2O7.

### Exemple 9 : synthèse de la (S)1-tert-butoxycarbonyl-3-(tert-butoxycarbonylamino)-3-isopropyl-3,4-dihydroquinoline-1(2H)-2,4-dione (I-k)

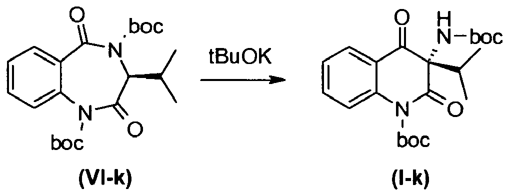

Le composé **(I-k)** a été obtenu sous la forme d'une huile incolore. Rendement 40 % (non-optimisé). [α]D20 = +17 (c= 0,87, CH₂Cl₂). ¹H-RMN (CDCl₃, 300 MHz) : δ 0,91 (d, 3H, CH₃CH, J = 6,9 Hz) ; 0,95 (d, 3H, CH₃CH, J = 6,9 Hz) ; 1,11 (s, 3H, CH₃C) ; 1,34 (s, 6H, CH₃C) ; 1,56, 1,59 (s, 9H, CH₃C) ; 2,12 - 2,24 (m, 1H, CHCH₃) ; 5,17 (s, 0,7H, NH) ; 5,21 (s, 0,3H, NH) ; 6,87 - 7,99 (m, 4H, Harom). ¹³C-RMN (CDCl₃, 75 MHz) : δ 16,9, 17,0 (CH₃CH) ; 27,5, 28,2 (CH₃C) ; 35,3, 36,0 (CH) ; 71,9, 72,1 (Cquat) ; 80,9, 82,0, 86,1, 86,6 (CCH₃) ; 114,7, 115,0, 123,9, 124,0, 135,7, 135,8 (CHarom); 120,2, 138,9 (Carom); 150,4, 153,8, 155,3 (CO carbamates); 169,5 (CO lactame) ; 191,6, 192,3 (CO cétone). Le dédoublement des signaux isopropyle (proton) et des groupes aromatiques/carbonyle (13C) était lié à un isomérisme E/Z sur le groupe Boc lactame. HPLCtr = 13,178 min. LC/MS : tr = 3,03 min : 419 [M+H⁺] ; 363 [(M+H⁺) - tBu] ; 319 [(M+H⁺) - Boc] ; 263 [(M+H⁺) - Boc - tBu]. HR-MS : 419,2173 Th (calculé 419,2182 Th) pour C22H30N2O6.

## Revendications

1. Procédé de synthèse d'un composé de formule (I) suivante : 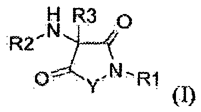 dans laquelle :
- R1 et R2 représentent indépendamment l'un de l'autre un groupe N-protecteur ;
- R3 représente un atome d'hydrogène, un groupe alkyle en C₁ à C₆, un groupe aryl(alkyle en C₁ à C₆) un groupe alcényle en C₂, à C₆, ou un groupe alkoxycarbonylalkyle ;
- Y représente
un groupe -C(HR4)- dans lequel R4 représente l'atome d'hydrogène, un groupe alkyle en C₁ à C₆, un groupe aryle, un groupe aryl(alkyle en C₁ à C₆), ou un groupe alcényle en C₂ à C₆ ; ou
un groupe *ortho*-phénylène,
**caractérisé en ce qu'**il comprend :
- une étape (a₁) consistant à faire réagir avec une base le composé de formule (II) suivante : 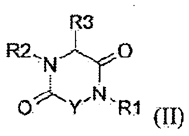
dans laquelle R1, R2, R3 et Y sont tels que définis précédemment, ou,
- une étape (a₂) consistant à faire réagir avec une base le composé de formule (III) suivante : 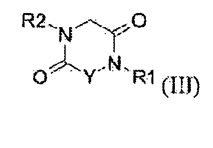 en présence d'un composé R3-X dans lesquelles R1 et R2 sont tels que définis précédemment, R3 est tel que défini précédemment à l'exclusion de l'hydrogène, et X représente un halogène,
ou,
- dans le cas où Y représente un groupe -C(HR4)-, une étape (a₃) consistant à faire réagir avec une base le composé de formule (IV) suivante : 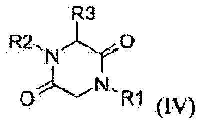
en présence d'un composé de formule R4-X dans lesquelles R1, R2, R3 sont tels que définis précédemment, R4 est tel que défini précédemment à l'exclusion de l'hydrogène, et X représente un halogène,
ou
- dans le cas où Y représente un groupe -C(HR4)-, une étape (a₄) consistant à faire réagir avec une base le composé de formule (V) suivante : 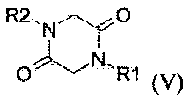
en présence d'un composé de formule R4-X, dans lesquelles R1 et R2 sont tels que définis précédemment, R3 est identique à R4 et est tel que défini précédemment à l'exclusion de l'hydrogène, et X représente un halogène.

2. Procédé selon la revendication 1, **caractérisé en ce que**, dans le cas où Y représente le groupe *ortho*-phénylène, l'étape (a₁) consiste à faire réagir avec une base le composé de formule (VI) suivante: 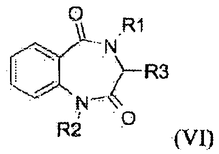 dans laquelle R1, R2 et R3 sont tels que définis dans la revendication 1.

3. Procédé selon l'une quelconque des revendications précédentes, **caractérisé en ce que** R1 et R2 peuvent être choisis indépendamment l'un de l'autre dans le groupe comprenant les alcoxycarbonyles et le benzoyle, de préférence le *tert-*butyloxycarbonyle ou le carbobenzyloxy.

4. Procédé selon l'une quelconque des revendications précédentes, **caractérisé en ce que** R3 et R4 peuvent être choisis indépendamment l'un de l'autre dans le groupe comprenant l'isopropyle, le benzyle, le méthyle, le *sec*-butyle, le prényle, l'allyle, - CH₂COOC₂H₅ et -CH₂CH₂COOCH₃.

5. Procédé selon l'une quelconque des revendications précédentes, **caractérisé en ce que** la base est choisie dans le groupe comprenant le *tert*-butylate de potassium, l'hydrure de sodium, la lithium diisopropylamine, le lithium hexaméthyldisilazane, le potassium hexaméthyldisilazane et le diméthylaminopyridine.

6. Procédé selon l'une quelconque des revendications précédentes, **caractérisé en ce que**, dans le cas où Y représente un groupe -C(HR4)- et R3 et R4 sont identiques et représentent un groupe allyle, il comprend une étape supplémentaire (b₁) consistant à faire subir au composé de formule (I) une réaction de métathèse des oléfines intramoléculaire pour donner le composé de formule (VII) suivante : 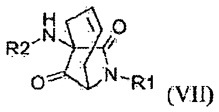 dans laquelle R1 et R2 sont tels que définis à la revendication 1.

7. Procédé selon la revendication 1, **caractérisé en ce que**, dans le cas où Y représente le groupe -C(HR4)-, il comprend les étapes successives supplémentaires suivantes consistant à :
- (b₂) faire réagir le composé de formule (I) en milieu basique avec un halogénure d'allyle pour donner le composé de formule (VIII) suivante : 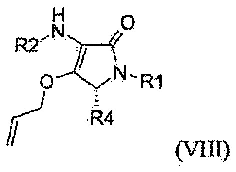 dans laquelle R1, R2 et R4 sont tels que définis à la revendication 1, de préférence R4 est l'isopropyle,
- (c) convertir par chauffage, de préférence aux micro-ondes, le composé de formule (VIII) en composé de formule (IX) suivante : 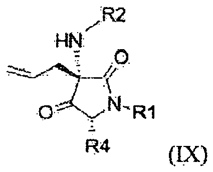
dans laquelle R1, R2 et R4 sont tels que définis la revendication 1, de préférence R4 est l'isopropyle,
- (d) faire réagir le composé de formule (IX) avec un halogénure d'allyle pour former le composé de formule (X) suivante : 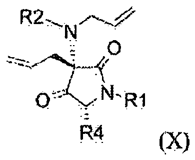
dans laquelle R1, R2 et R4 sont tels que définis à la revendication 1, de préférence R4 est l'isopropyle, et
- (e) convertir le composé de formule (X) par métathèse des oléfines intramoléculaire en composé de formule (XI) suivante : 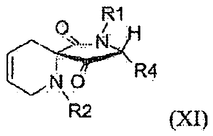
dans laquelle R1, R2 et R4 sont tels que définis à la revendication 1, de préférence R4 est l'isopropyle,

8. Procédé selon la revendications 1, **caractérisé en ce que**, dans le cas où R3 représente le groupe EtOOCCH₂- et Y représente le groupe -C(HR4)-, il comprend une étape supplémentaire (b₃) consistant à faire réagir le composé de formule (I) avec un hydrure pour former le composé de formule (XII) suivante : 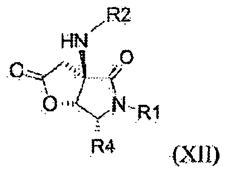 dans laquelle R1, R2 et R4 sont tels que définis à la revendication 1, de préférence R4 est l'isopropyle.

9. Procédé selon la revendications 1, **caractérisé en ce que**, lorsque Y représente un groupe -(CHR4)-, il comprend une étape supplémentaire (b₄) consistant à faire réagir le composé de formule (I) avec l'acide trifluoroacétique à une concentration massique comprise entre 3% et 10%, de préférence égale à 5% pour donner le composé de formule (XIII) suivante : 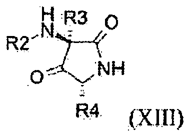 dans laquelle R2, R3 et R4 sont tels que définis à la revendication 1, de préférence R4 est l'isopropyle.

10. Procédé selon la revendication 9, **caractérisé en ce qu'**il comprend une étape supplémentaire (C₄) consistant à faire réagir le composé de formule (XIII) avec un hydrure, de préférence NaBH₄, pour former le composé de formule (XIV) suivante : 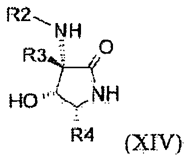 dans laquelle R2, R3 et R4 sont tels que définis à la revendication 1, de préférence R4 est l'isopropyle.

11. Procédé selon la revendication 9, **caractérisé en ce qu'**il comprend une étape supplémentaire (c₅) consistant à faire réagir le composé de formule (XIII) avec l'acide trifluoroacétique à une concentration massique comprise entre 10% et 25%, de préférence égale à 15% pour donner le composé de formule (XV) suivante : 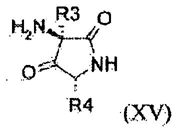 dans laquelle R3 et R4 sont tels que définis à la revendication 1, de préférence R4 est l'isopropyle.

12. Procédé selon la revendications 1, **caractérisé en ce que**, dans le cas où Y représente le groupe -(CHR4)-, il comprend une étape supplémentaire (b₅) consistant à faire réagir le composé de formule (I) avec un borohydrure, de préférence NaBH₄, pour former le composé de formule (XVI) suivante : 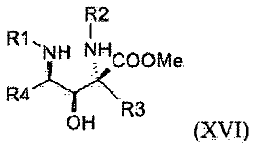 dans laquelle R1, R2, R3 et R4 sont tels que définis à la revendication 1.

## Claims

1. Method for synthesizing a compound having the following formula (I)):
in which: 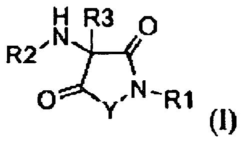
- R1 and R2 are each independently an N-protecting group;
- R3 is a hydrogen atom, a C₁ to C₆ alkyl group, a C₁ to C₆ arylalkyl group, a C₂ to C₆ alkenyl group or an alcoxycarbonylalkyl group;
- Y is a -C(HR4)- group in which R4 is the hydrogen atom, a C₁ to C₆ alkyl group, an aryl group, a C₁ to C₆ arylalkyl group or a C₂ to C₆ alkenyl group; or an ortho-phenylene group,
**characterized in that** it comprises:
- a step (a₁) consisting of reacting, with a base, the compound having the following formula (II): 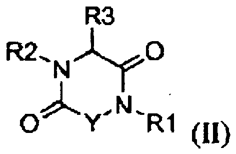 in which R1, R2, R3 and Y are as previously defined, or
- a step (a₂) consisting of reacting, with a base, the compound having the following formula (III): 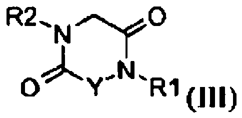 in the presence of a compound R3-X in which R1 and R2 are as defined previously, R3 is as defined previously with the exclusion of hydrogen, and X is a halogen,
or,
- if Y is a -C(HR4)- group, a step (a₃) consisting of reacting the compound having the following formula (IV) with a base: 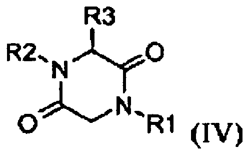 in the presence of a compound having formula R4-X in which R1, R2, R3 are as previously defined, R4 is as previously defined with the exclusion of hydrogen, and X is a halogen,
or
- if Y is a -C(HR4)- group, a step (a₄) consisting of reacting, with a base, the compound having the following formula (V): 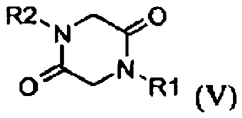 in the presence of a compound having formul²
a R4-X, in which R1 and R2 are as previously defined, R3 is identical to R4 and is as defined previously with the exclusion of hydrogen, and X is a halogen.

2. Method according to claim 1, **characterized in that**, if Y is the ortho-phenylene group, step (a₁) consists of reacting, with a base, the following compound having formula (VI): 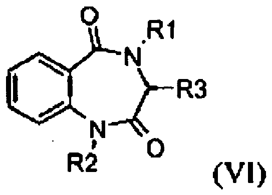 in which R1, R2 and R3 are as defined in claim 1.

3. Method according to any of the preceding claims, **characterized in that** R1 and R2 may each be chosen independently from the group comprising alcoxycarbonyls and benzoyl, preferably tert-butyloxycarbonyl or carbobenzyloxy.

4. Method according to any of the preceding claims, **characterized in that** R3 and R4 may each be chosen independently from the group comprising isopropyl, benzyl, methyl, sec-butyl, prenyl, allyl, -CH₂COOC₂H₅ and -CH₂CH₂COOCH₃.

5. Method according to any of the preceding claims, **characterized in that** the base is chosen from the group comprising potassium tert-butylate, sodium hydride, lithium diisopropylamine, lithium hexamethyldisilazane, potassium hexamethyldisilazane and dimethylaminopyridine.

6. Method according to any of the preceding claims, **characterized in that**, if Y is a -C-(HR4)- group and R3 and R4 are identical and are an allyl group, it comprises an additional step (b₁) consisting of submitting the compound having formula (I) to an intramolecular olefin metathesis reaction to give the following compound having formula (VII): 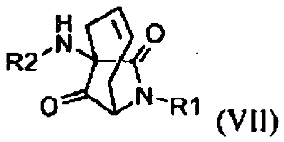 in which R1 and R2 are as defined in claim 1.

7. Method according to claim 1, **characterized in that** if Y is the -C(HR4)- group, it comprises the following additional successive steps consisting of:
- (b₂) reacting the compound having formula (I) in a basic medium with an allyl halide to give the following compound (VIII): 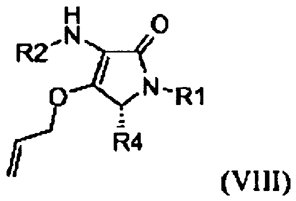 in which R1, R2 and R4 are as defined in claim 1, preferably R4 is isopropyl,
- (c) converting by heating, preferably with microwaves, the compound having formula (VIII) into the following compound having formula (IX): 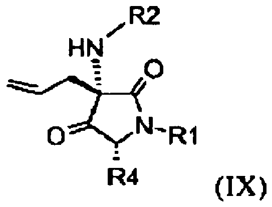 in which R1, R2 and R4 are as defined in claim 1, preferably R4 is isopropyl,
- (d) reacting the compound having formula (IX) with an allyl halide to form the following compound having formula (X): 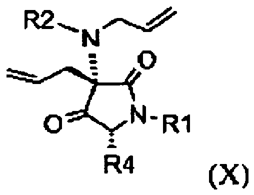 in which R1, R2 and R4 are as defined in claim 1, preferably R4 is isopropyl, and
- (e) converting the compound having formula (X,) by intramolecular olefin metathesis, into the following compound having formula (XI): 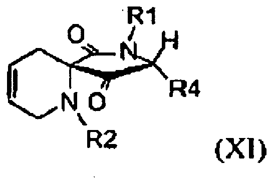 in which R1, R2 and R4 are as defined in claim 1, preferably R4 is isopropyl.

8. Method according to claim 1 **characterized in that**, if R3 is the EtOOCCH₂ group and Y is the - C(HR4)- group, it comprises an additional step (b₃) consisting of reacting the compound having formula (I) with a hydride, to form the following compound having formula (XII): 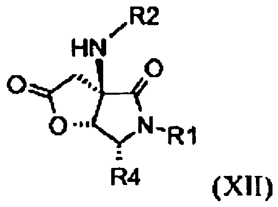 in which R1, R2 and R4 are as defined in claim 1, preferably R4 is isopropyl.

9. Method according to claim 1 **characterized in that**, if Y is a -(CHR4)- group, it comprises an additional step (b₄) consisting of reacting the compound having formula (I) with trifluoroacetic acid at a weight concentration of between 3% and 10%, preferably 5%, to give the following compound having formula (XIII): 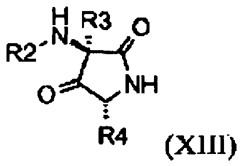 in which R2, R3 and R4 are as defined in claim 1, preferably R4 is isopropyl.

10. Method according to claim 9 **characterized in that** it comprises an additional step (c₄) consisting of reacting the compound having formula (XIII) with a hydride, preferably NaBH₄ to form the following compound having formula (XIV) : 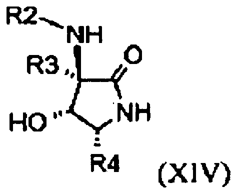 in which R2, R3 and R4 are as defined in claim 1, preferably R4 is isopropyl.

11. Method according to claim 9 **characterized in that** it comprises an additional step (c₅) consisting of reacting the compound having formula (XIII) with trifluoroacetic acid at a weight concentration of between 10% and 25%, preferably 15%, to give the following compound having formula (XV): 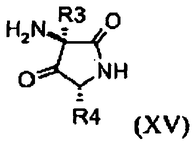 in which R3 and R4 are as defined in claim 1, preferably R4 is isopropyl.

12. Method according to claim 1, **characterized in that** if Y is the -(CHR4)- group, it comprises an additional step (b₅) consisting of reacting the compound having formula (I) with a borohydride, preferably NaBH₄, to form the following compound having formula (XVI): 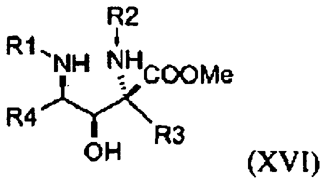 in which R1, R2, R3 and R4 are as defined in claim 1.

## Patentansprüche

1. Verfahren zur Synthese einer Verbindung der folgenden Formel (I): 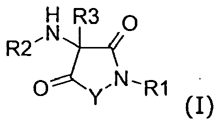 wobei:
- R1 und R2 unabhängig voneinander eine N-Schutzgruppe darstellen;
- R3 ein Wasserstoffatom, eine C₁- bis C₆-Alkylgruppe, eine Aryl(C₁-bis C₆-Alkyl)-Gruppe, eine C₂- bis C₆-Alkenylgruppe oder eine Alkoxycarbonylalkylgruppe darstellt;
- Y
eine -C(HR4)-Gruppe darstellt, wobei R4 das Wasserstoffatom, eine C₁- bis C₆-Alkylgruppe, eine Arylgruppe, eine Aryl(C₁- bis C₆-Alkyl)-Gruppe oder eine C₂- bis C₆-Alkenylgruppe darstellt; oder
eine ortho-Phenylengruppe darstellt,
**dadurch gekennzeichnet, dass** es Folgendes umfasst:
- einen Schritt (a₁), der darin besteht, die Verbindung der folgenden Formel (II) : 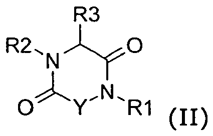 mit einer Base reagieren zu lassen, wobei R1, R2, R3 und Y wie vorstehend definiert sind,
oder
- einen Schritt (a₂), der darin besteht, die Verbindung der folgenden Formel (III) : 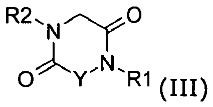 in Gegenwart einer Verbindung R3-X mit einer Base reagieren zu lassen, wobei R1 und R2 wie vorstehend definiert sind, R3 wie vorstehend definiert ist, unter Ausschluss von Wasserstoff, und X ein Halogen darstellt,
oder
- für den Fall, dass Y eine -C(HR4)-Gruppe darstellt, einen Schritt (a₃), der darin besteht, die Verbindung der folgenden Formel (IV) : 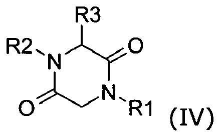 in Gegenwart einer Verbindung der Formel R4-X mit einer Base reagieren zu lassen, wobei R1, R2, R3 wie vorstehend definiert sind, wobei R4 wie vorstehend definiert ist, unter Ausschluss von Wasserstoff, und X ein Halogen darstellt,
oder
- für den Fall, dass Y eine -C(HR4)-Gruppe darstellt, einen Schritt (a₄), der darin besteht, die Verbindung der folgenden Formel (V) : 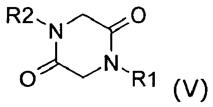 ein Gegenwart einer Verbindung der Formel R4-X mit einer Base reagieren zu lassen, wobei R1 und R2 wie vorstehend definiert sind, R3 mit R4 identisch ist und wie vorstehend definiert ist, unter Ausschluss von Wasserstoff, und X ein Halogen darstellt.

2. Verfahren nach Anspruch 1, **dadurch gekennzeichnet, dass**, für den Fall, dass Y die Ortho-Phenylengruppe darstellt, der Schritt (a₁) darin besteht, die Verbindung der folgenden Formel (VI) : 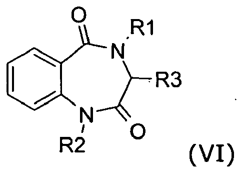 mit einer Base reagieren zu lassen, wobei R1, R2 und R3 wie in Anspruch 1 definiert sind.

3. Verfahren nach einem der vorhergehenden Ansprüche, **dadurch gekennzeichnet, dass** R1 und R2 unabhängig voneinander aus der Gruppe umfassend Alkoxycarbonyle und Benzoyl, vorzugsweise tert-Butyloxycarbonyl oder Carbobenzyloxy, gewählt sein können.

4. Verfahren nach einem der vorhergehenden Ansprüche, **dadurch gekennzeichnet, dass** R3 und R4 unabhängig voneinander aus der Gruppe umfassend Isopropyl, Benzyl, Methyl, sec-Butyl, Prenyl, Allyl, -CH₂COOC₂H₅ und -CH₂CH₂COOCH₃ gewählt sein können.

5. Verfahren nach einem der vorhergehenden Ansprüche, **dadurch gekennzeichnet, dass** die Base aus der Gruppe umfassend Kalium-tert-butylat, Natriumhydrid, Lithium-Diisopropylamin, Lithium-Hexamethyldisilazan, Kalium-Hexamethyldisilazan und Dimethylaminopyridin gewählt ist.

6. Verfahren nach einem der vorhergehenden Ansprüche, **dadurch gekennzeichnet, dass**, für den Fall, dass Y eine -C(HR4)-Gruppe darstellt und R3 und R4 identisch sind und eine Allylgruppe darstellen, es einen zusätzlichen Schritt (b₁) umfasst, der darin besteht, die Verbindung der Formel (I) einer intramolekularen Olefinmetathesereaktion zu unterziehen, um die Verbindung der folgenden Formel (VII) zu bilden: 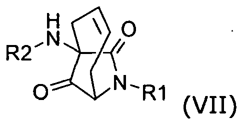 wobei R1 und R2 wie in Anspruch 1 definiert sind.

7. Verfahren nach Anspruch 1, **dadurch gekennzeichnet, dass**, für den Fall, dass Y die -C(HR4)-Gruppe darstellt, es die folgenden zusätzlichen, aufeinander folgenden Schritte umfasst, die darin bestehen:
- (b₂) die Verbindung der Formel (I) in basischem Medium mit einem Allylhalogenid reagieren zu lassen, um die Verbindung der folgenden Formel (VIII) zu bilden: 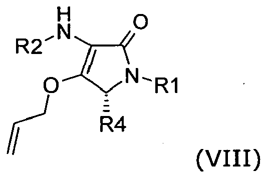 wobei R1, R2 und R4 wie in Anspruch 1 definiert sind, wobei R4 vorzugsweise Isopropyl ist,
- (c) die Verbindung der Formel (VIII) durch Erhitzen, vorzugsweise durch Mikrowellen, in die Verbindung der folgenden Formel (IX) umzuwandeln: 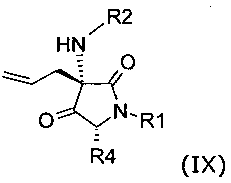 wobei R1, R2 und R4 wie in Anspruch 1 definiert sind, wobei R4 vorzugsweise Isopropyl ist,
- (d) die Verbindung der Formel (IX) mit einem Allylhalogenid reagieren zu lassen, um die Verbindung der folgenden Formel (X) zu bilden: 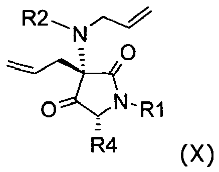 wobei R1, R2 und R4 wie in Anspruch 1 definiert sind, wobei R4 vorzugsweise Isopropyl ist, und
- (e) die Verbindung der Formel (X) durch intramolekulare Olefinmetathese in die Verbindung der folgenden Formel (XI) umzuwandeln: 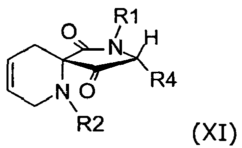 wobei R1, R2 und R4 wie in Anspruch 1 definiert sind, wobei R4 vorzugsweise Isopropyl ist.

8. Verfahren nach Anspruch 1, **dadurch gekennzeichnet, dass**, für den Fall, dass R3 die EtOOCCH₂-Gruppe und Y die -C(HR4)-Gruppe darstellt, es einen zusätzlichen Schritt (b₃) umfasst, der darin besteht, die Verbindung der Formel (I) mit einem Hydrid reagieren zu lassen, um die Verbindung der folgenden Formel (XII) zu bilden: 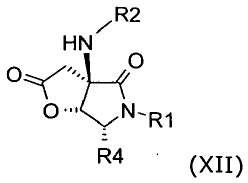 wobei R1, R2 und R4 wie in Anspruch 1 definiert sind, wobei R4 vorzugsweise Isopropyl ist.

9. Verfahren nach Anspruch 1, **dadurch gekennzeichnet, dass**, wenn Y eine -(CHR4)-Gruppe darstellt, es einen zusätzlichen Schritt (b₄) umfasst, der darin besteht, die Verbindung der Formel (I) mit Trifluoressigsäure in einer Massenkonzentration zwischen 3% und 10%, vorzugsweise gleich 5%, reagieren zu lassen, um die Verbindung der folgenden Formel (XIII) zu bilden: 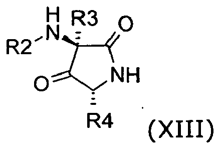 wobei R2, R3 und R4 wie in Anspruch 1 definiert sind, wobei R4 vorzugsweise Isopropyl ist.

10. Verfahren nach Anspruch 9, **dadurch gekennzeichnet, dass** es einen zusätzlichen Schritt (c₄) umfasst, der darin besteht, die Verbindung der Formel (XIII) mit einem Hydrid, vorzugsweise NaBH₄, reagieren zu lassen, um die Verbindung der folgenden Formel (XIV) zu bilden: 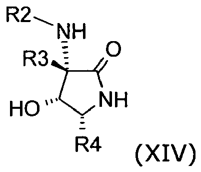 wobei R2, R3 und R4 wie in Anspruch 1 definiert sind, wobei R4 vorzugsweise Isopropyl ist.

11. Verfahren nach Anspruch 9, **dadurch gekennzeichnet, dass** es einen zusätzlichen Schritt (c₅) umfasst, der darin besteht, die Verbindung der Formel (XIII) mit Trifluoressigsäure in einer Massenkonzentration zwischen 10% und 25%, vorzugsweise gleich 15%, reagieren zu lassen, um die Verbindung der folgenden Formel (XV) zu bilden: 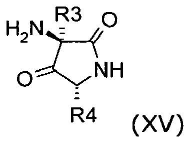 wobei R3 und R4 wie in Anspruch 1 definiert sind, wobei R4 vorzugsweise Isopropyl ist.

12. Verfahren nach Anspruch 1, **dadurch gekennzeichnet, dass**, für den Fall, dass Y die -(CHR4)-Gruppe darstellt, es einen zusätzlichen Schritt (b₅) umfasst, der darin besteht, die Verbindung der Formel (I) mit einem Borhydrid, vorzugsweise NaBH₄, reagieren zu lassen, um die Verbindung der folgenden Formel (XVI) zu bilden: 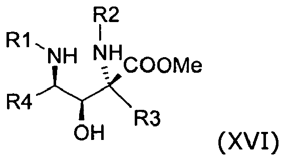 wobei R1, R2, R3 und R4 wie in Anspruch 1 definiert sind.
